(19) Europäisches Patentamt
European Patent Office
Office européen des brevets

(11) EP 4 368 627 A1

(12) **EUROPEAN PATENT APPLICATION**
published in accordance with Art. 153(4) EPC

(43) Date of publication:
**15.05.2024 Bulletin 2024/20**

(21) Application number: **22837536.6**

(22) Date of filing: **28.06.2022**

(51) International Patent Classification (IPC):
***C07H 21/02*** *(2006.01)*

(52) Cooperative Patent Classification (CPC):
**C07H 21/02;** Y02P 20/55

(86) International application number:
**PCT/JP2022/025692**

(87) International publication number:
**WO 2023/282120 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.07.2021 JP 2021112316**

(71) Applicant: **Sumitomo Chemical Company, Limited**
**Tokyo 103-6020 (JP)**

(72) Inventor: **MORIYAMA, Yuya**
**Osaka-shi, Osaka 555-0021 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

(54) **COMPOSITION CONTAINING NUCLEIC ACID OLIGOMER**

(57) The present invention provides a stable composition containing a nucleic acid oligomer having a phosphorothioate bond, a method for producing the same, and a method for efficiently producing the nucleic acid oligomer from the composition. The composition containing a nucleic acid oligomer having a phosphorothioate bond represented by Formula (1) (wherein symbols are as defined in the specification), an alkyl ammonium salt, a nitrile-based organic solvent, water, and an additive containing at least one compound selected from the group consisting of compounds represented by Formulae (3a) to (3h) is a stable composition.

EP 4 368 627 A1

## Description

### TECHNICAL FIELD

The present invention relates to a composition containing a nucleic acid oligomer. More specifically, the present invention relates to a composition containing a nucleic acid oligomer containing phosphorothioate.

### BACKGROUND ART

In recent years, there has been growing interest in the application of a nucleic acid oligomer to the medical field. Examples thereof include an antisense nucleic acid, an aptamer, a ribozyme, and nucleic acids that induces RNA interference (RNAi) such as siRNA, which are referred to as a nucleic acid medicine.

The nucleic acid oligomer has been known to be synthesized by a solid phase synthesis method, and a nucleic acid oligomer having a phosphorothioate bond has also been known as a useful compound synthesized by a solid phase method (Patent Document 1).

### PRIOR ART DOCUMENT

### PATENT DOCUMENT

Patent Document 1: WO 2017/068377 A1

### SUMMARY OF THE INVENTION

### PROBLEMS TO BE SOLVED BY THE INVENTION

The nucleic acid oligomer having a phosphorothioate bond may have any problem of stability in its production process. An object of the present invention is to provide a stable composition containing a nucleic acid oligomer having a phosphorothioate bond, a method for producing the same, and a method for efficiently producing the nucleic acid oligomer from the composition.

### MEANS FOR SOLVING THE PROBLEMS

As a result of intensive studies to achieve the above object, the present inventor has found that a composition obtained by mixing a nucleic acid oligomer obtained by subjecting a crude product of a nucleic acid oligomer having a phosphorothioate bond, which is produced by a phosphoramidite method in a solid phase synthesis method, to a reverse phase chromatography treatment, with an alkyl ammonium salt, a nitrile-based organic solvent, water, and a certain additive can be stabilized. Therefore, the present invention provides the composition, a method for producing the composition, and a method for efficiently producing a nucleic acid oligomer from the composition.

The present invention includes, but is not limited to, the following aspects.

1. A composition comprising a nucleic acid oligomer having a phosphorothioate bond represented by Formula (1):

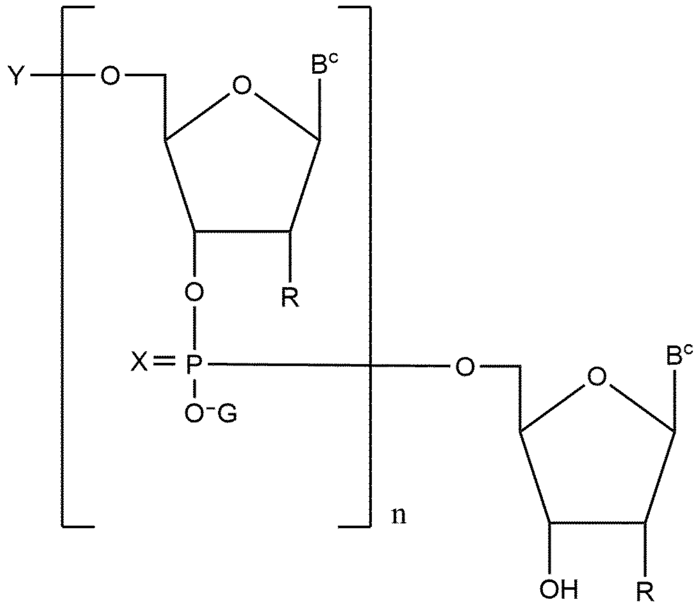

(1)

wherein:

$B^C$ each independently represents the same nucleobase or different nucleobases,
R is the same or different from each other and each independently represents a hydrogen atom, a fluorine atom, or OQ group,
Q is the same or different from each other and each independently represents a hydrogen atom, a methyl group, a 2-methoxyethyl group, a methylene group bonded to a carbon atom at a 4'-position of ribose, an ethylene group bonded to a carbon atom at a 4'-position of ribose, or an ethylidene group bonded to a carbon atom at a 4'-position of ribose,
X is the same or different from each other and each independently represents an oxygen atom or a sulfur atom, provided that at least one X represents a sulfur atom,
Y represents a hydrogen atom or a protecting group of a hydroxy group,
G represents an ammonium ion, an alkylammonium ion, an alkali metal ion, a hydrogen ion or a hydroxyalkylammonium ion, and
n is an integer satisfying Formula (2):

$$15 \leq n \quad (2),$$

an alkyl ammonium salt, a nitrile-based organic solvent, water, and an additive,
wherein the additive is an additive containing at least one compound selected from the group consisting of compounds represented by the following Formulae (3a) to (3h) :

Formula (3a) : $P(L^1)_3$,

Formula (3b): $P(OL^1)_3$,

Formula (3c) : $P(L^1)_2(OL^1)$,

Formula (3d) : $PL(OL^1)_2$,

Formula (3e): $PH(O)(OL^1)_2$,

Formula (3f): $PH(O)L^1(OL^1)$,

Formula (3g) : $(P(L^1)_2)_2-L^2$,

Formula (3h):

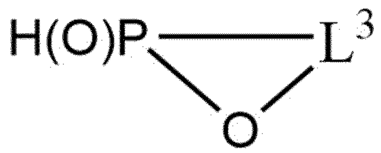

,

wherein, in each of Formulae (3a) to (3h), $L^1$ is the same or different from each other and each independently represent a C6-10 aryl group which may be substituted with 1 to 3 substituents selected from the group consisting of a C1-6 alkyl group and a C1-6 alkoxy group, or a C1-6 alkyl group,

$L^2$ is the same or different from each other and each independently represents a C6-14 arylene group which may be substituted with 1 to 3 substituents selected from the group consisting of a C1-6 alkyl group and a C1-6 alkoxy group, or a C1-6 alkylene group, and

$L^3$ is the same or different from each other and each independently represents a C6-14 arylene group which may be substituted with 1 to 3 substituents selected from the group consisting of a C1-6 alkyl group and a C1-6 alkoxy group.

2. The composition according to the item 1, wherein the additive is at least one compound selected from the group consisting of triphenylphosphine, diethyl phosphite, triethyl phosphite, diethoxyphenylphosphine, ethoxydiphenylphosphine, and 9,10-dihydro-9-oxa-10-phosphaphenanthrene 10-oxide.
3. The composition according to any one of the items 1 and 2, wherein the alkyl ammonium salt is at least one alkyl ammonium salt selected from the group consisting of a monoalkylammonium salt and a dialkylammonium salt.
4. The composition according to any one of the items 1 to 3, wherein the nitrile-based organic solvent is acetonitrile.
5. The composition according to any one of the items 1 to 4, wherein in Formula (1), R is each independently a hydroxy group or a methoxy group.
6. A method for producing a nucleic acid oligomer, comprising mixing the composition according to any one of the items 1 to 5 with at least one solvent selected from the group consisting of a C1-4 alcohol, tetrahydrofuran and dioxane to isolate a precipitated nucleic acid oligomer.
7. A method for producing the composition according to any one of the items 1 to 5, comprising mixing a column eluate containing a nucleic acid oligomer represented by Formula (1) wherein the nucleic acid oligomer is obtained by subjecting a crude product of the nucleic acid oligomer of Formula (1) synthesized by a solid phase synthesis method to a reverse phase column chromatography treatment, an alkyl ammonium salt, a water-soluble organic solvent, and water, with an additive.

EFFECT OF THE INVENTION

The present invention provides a stable composition containing a nucleic acid oligomer having a phosphorothioate bond, and a method for efficiently producing the nucleic acid oligomer using the same.

BRIEF DESCRIPTION OF THE DRAWINGS

Fig. 1 is a chart showing an example of synthesis of a nucleic acid oligomer by a phosphoramidite method.

MODE FOR CARRYING OUT THE INVENTION

A composition containing a nucleic acid oligomer having a phosphorothioate bond represented by Formula (1), an alkyl ammonium salt, a nitrile-based organic solvent, water, and an additive, wherein the additive is at least one compound selected from the group consisting of a compound having a disulfide bond and a compound having a sulfide bond will be described.

In Formula (1), a nucleobase represented by $B^C$ (hereinafter, may be referred to as "base") may be a natural or non-natural nucleobase. Examples of the non-natural nucleobase include modified analogs of natural or non-natural nucleobases. Typical examples of the nucleobase include a purine compound and a pyrimidine compound, and include, for example, nucleobases disclosed in US 3,687,808 A, "Concise Encyclopedia of Polymer Science and Engineering", pp. 858-859, edited by Kroschwitz J. I., John Wiley & Sons, 1990, and Englisch et al., Angewandte Chemie, International Edition, 1991, vol. 30, p. 613.

Specific examples of the nucleobase include purine bases such as adenine, isoguanine, xanthine, hypoxanthine, and guanine; and pyrimidine bases such as cytosine, uracil, and thymine.

Furthermore, examples of the nucleobase represented by $B^C$ include amino derivatives such as 2-aminoadenine, 2-aminopurine, and 2,6-diaminopurine; alkyl derivatives such as 5-methyluracil, 5-methylcytosine, 7-methylguanine, 6-methylpurine, and 2-propylpurine; 5-halouracil and 5-halocytosine; 5-propynyluracil and 5-propynylcytosine; 6-azauracil, 6-azacytosine, and 6-azathimine; 5-uracil(pseudouracil), 4-thiouracil, 5-(2-aminopropyl)uracil, and 5-aminoallyluracil; 8-substituted purines, for example, 8-halogenated, aminated, thiolated, thioalkylated or hydroxylated purine, and other 8-substituted purine; 5-trifluoromethylated pyrimidine, and other 5-substituted pyrimidine; 6-azapyrimidine; N-2, N-6 and O-6 substituted purines (including 2-aminopropyl adenine); dihydrouracil; 3-deaza-5-azacytosine; 7-deazaadenine; N6-methyladenine, N6,N6-dimethyladenine; 5-amino-allyl-uracil; N3-methyluracil; substituted 1,2,4-triazole; 2-pyridinone; 5-nitroindole; 3-nitropyrrole; 5-methoxyuracil; uacil-5-oxyacetic acid; 5-methoxycarbonylmethyluracil; 2-thiouracil, 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; N4-acetylcytosine; 2-thiocytosine; N6-methyladenine; N6-isopentyladenine; 2-methylthio-N6-isopentenyladenine; N-methylguanine; and O-alkylated bases.

When R represents OQ group, and Q represents a methylene group bonded to a carbon atom at the 4'-position of ribose, an ethylene group bonded to a carbon atom at the 4'-position of ribose, or an ethylidene group bonded to a carbon atom at the 4'-position of ribose, the structures are represented by LNA-1, LNA-2, and LNA-3 structures represented by the following Formulae (3).

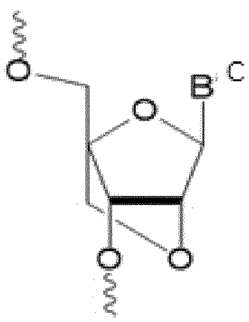
LNA-1

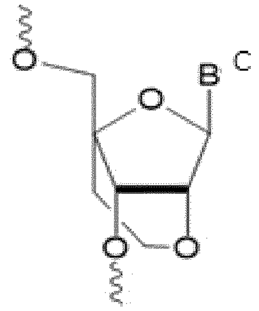
LNA-2

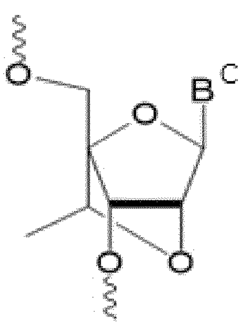
LNA-3

(3)

(wherein $B^c$ represents a nucleobase as described above) .

A protecting group of a hydroxyl group represented by Y can be used without particular limitation as long as it can function as a protecting group in an amidite method, and for example, a known protecting group used for an amidite compound can be widely used. The protecting group of the hydroxyl group represented by Y is preferably the following group.

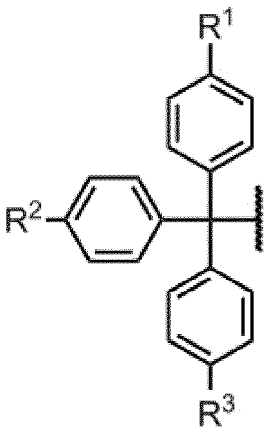

(wherein $R^1$, $R^2$, and $R^3$ are the same or different from each other and each independently represent a hydrogen or an alkoxy group).

Examples of the alkoxy group include a methoxy group.

The chain length of the nucleic acid oligomer of Formula (1) is $n \geq 15$. Examples of the upper limit of the chain

length include $n \leq 200$. In the nucleic acid oligomer, at least one of n Xs may be a sulfur atom, and all Xs may be sulfur atoms. For example, in the case of n=103, examples of the number of the sulfur atoms include 6, 12, or 20.

The nucleic acid oligomer of Formula (1) may be, for example, a DNA or RNA oligomer, or an oligomer thereof containing a non-natural nucleobase. The nucleic acid oligomer is typically a single-stranded DNA or RNA oligomer. In the nucleic acid oligomer of Formula (1), preferably, the substituents R each independently represent a hydroxy group or a methoxy group. The nucleic acid oligomer is preferably RNA which is the nucleic acid oligomer of Formula (1), wherein the substituents R each independently represent a hydroxy group or a methoxy group. More specifically, the nucleic acid oligomer preferably contains both a nucleotide having a hydroxy group as the substituent R and a nucleotide having a methoxy group as the substituent R.

The concentration of the nucleic acid oligomer in the composition is usually 0.05 mg/mL to 5 mg/mL, preferably 0.05 mg/mL to 1 mg/mL, and more preferably 0.1 mg/mL to 0.5 mg/mL.

As the alkylammonium salt, a monoalkylammonium salt, a dialkylammonium salt, and a trialkylammonium salt are usually used, a monoalkylammonium salt and a dialkylammonium salt are preferably used, and a dialkylammonium salt is more preferably used. The number of carbon atoms of a monoalkylamine forming the monoalkylammonium salt is preferably 3 to 10, and more preferably 4 to 6. The monoalkylamine is still more preferably hexylamine. The number of carbon atoms of a dialkylamine forming the dialkylammonium salt is preferably 4 to 10 and more preferably 5 to 9. The dialkylamine is preferably di-n-butylamine. A trialkylamine forming the trialkylammonium salt preferably has 6 to 12 carbon atoms, and more preferably 6 to 9 carbon atoms. Specific examples of the trialkylamine include triethylamine.

Examples of acids forming the monoalkylammonium salt, the dialkylammonium salt, and the trialkylammonium salt include carbonic acid, acetic acid, formic acid, trifluoroacetic acid, and propionic acid.

The concentration of the ammonium salt is usually 1 to 200 mM, preferably 5 to 150 mM, and more preferably 20 to 100 mM.

Examples of the nitrile-based organic solvent include acetonitrile. The amount of the nitrile-based organic solvent in the composition is usually 10 to 70%, preferably 20 to 60%, and more preferably 30 to 50%, based on the total mass of the composition (herein "%" represents % by mass) .

The composition may further contain an alcohol-based organic solvent. Examples of the alcohol-based organic solvent include a C1-4 alcohol, and the alcohol-based organic solvent is preferably a C1-3 alcohol, more preferably a C1-2 alcohol, and still more preferably methanol. The amount of the alcoholic organic solvent in the composition is usually 0 to 20%, preferably 0 to 15%, and more preferably 0% to 10%, based on the total mass of the composition (herein "%" represents % by mass).

The amount of water may be any amount that balances to satisfy the concentration range of each of the components, and is usually 90% to 30%, preferably 80% to 40%, and more preferably 70% to 40%, based on the total mass of the composition (herein "%" represents % by mass).

At least one compound selected from the group consisting of compounds represented by Formulae (3a) to (3h) will be described.

Examples of a C1-6 alkyl group in a C6-10 aryl group which may be substituted with 1 to 3 substituents selected from the group consisting of a C1-6 alkyl group and a C1-6 alkoxy group, represented by $L^1$ include a methyl group, an ethyl group, a propyl group, a butyl group, a pentyl group, and a hexyl group, and in the alkyl group, a C1-4 alkyl group is more preferable, and a C1-2 alkyl group is still more preferable. Examples of the C1-6 alkoxy group include a methoxy group, an ethoxy group, a propoxy group, a butoxy group, a pentyloxy group, and a hexyloxy group. Examples of the C6-10 aryl group include a phenyl group, a 1-naphthyl group, and a 2-naphthyl group, and a phenyl group is preferable. Examples of the C6-10 aryl group substituted with 1 to 3 substituents selected from the group consisting of a C1-6 alkyl group and a C1-6 alkoxy group include a tolyl group and a methoxyphenyl group.

Specific examples of the compound represented by Formula (3a) include triphenylphosphine.

Examples of the C1-6 alkyl group represented by $L^1$ include the same groups as described above. Specific examples of the compound represented by Formula (3b) include triethyl phosphite.

Specific examples of the compound represented by Formula (3c) include ethoxydiphenylphosphine.

Specific examples of the compound represented by Formula (3d) include diethoxyphenylphosphine.

Specific examples of the compound represented by Formula (3e) include diethyl phosphite.

Specific examples of the compound represented by Formula (3f) include ethyl phenylphosphinate.

Examples of a C6-14 arylene group which may be substituted with 1 to 3 substituents selected from the group consisting of a C1-6 alkyl group and a C1-6 alkoxy group, represented by $L^2$ include a 1,2-phenylene group, a 1,8-naphthylene group, and a 1,6-biphenylene group.

Examples of a C1-6 alkylene group represented by $L^2$ include a methylene group, an ethylene group, a propylene group, a butylene group, a pentylene group, and a hexylene group.

Examples of a C6-14 arylene group which may be substituted with 1 to 3 substituents selected from the group consisting of a C1-6 alkyl group and a C1-6 alkoxy group, represented by $L^3$ include the same groups as those exemplified for $L^2$ described above.

Specific examples of the compound represented by Formula (3g) include 1,2-bis(diphenylphosphino)benzene, 1,8-bis((diphenylphosphino)naphthalene, and 2,2'-bis(diphenylphosphino)biphenyl.

Specific examples of the compound represented by Formula (3h) include 9,10-dihydro-9-oxa-10-phosphaphenanthrene 10-oxide.

The concentration of the additive is usually 0.1 μM to 100 mM, and preferably 1 mM to 10 mM.

The composition of the present invention is usually obtained by adding the above-described additive to a column eluate obtained by subjecting a crude product of the nucleic acid oligomer of Formula (1) synthesized by a solid phase synthesis method to a reverse phase column chromatography treatment using a mobile phase containing an alkyl ammonium salt, a water-soluble organic solvent, and water. Alternatively, the composition of the present invention may be prepared as an elution fraction of reverse phase column chromatography by using the mobile phase containing the additive in advance. Here, the water-soluble organic solvent means an organic solvent containing the nitrile-based water-soluble organic solvent and, as appropriate, a hydrophilic organic solvent (for example, the alcohol-based organic solvent) usually known in the organic chemistry field.

The eluted fraction obtained by reverse phase column chromatography is analyzed on its constitution with UV absorption at a wavelength of 260 nm under the condition of chromatography generally used for separation analysis of nucleic acid, and is then selected and collected. A nucleic acid oligomer having a predetermined amount of phosphorothioate bonds, which is a target product, is obtained from the collected fraction. As the analysis method, for example, a method described in Non-Patent Document (Handbook of Analysis of Oligonucleotides and Related Products, CRC Press) can be used.

Examples of a filler for reverse phase column chromatography include silica or a polymer in which any one or more selected from a phenyl group, an alkyl group having 1 to 20 carbon atoms, and a cyanopropyl group are immobilized as silica or a polymer serving as a hydrophobic stationary phase. Examples of the silica or the polymer to be used as such a filler include those having a particle diameter of 2 um or more, or 5 um or more.

As a mobile phase of the reverse phase column chromatography, for example, a mobile phase containing an aqueous solution of an ammonium salt having a concentration and a pH as described above and a mobile phase containing the water-soluble organic solvent and used by applying a gradient for sequentially increasing the concentration are used. The temperature of the reverse phase column chromatography is usually 20 to 100°C, preferably 30 to 80°C, and more preferably 40 to 70°C. The composition of the present invention is typically obtained as the eluent fraction of the reverse phase column chromatography as described above.

For example, after a storage step, the composition of the present invention may be subjected to a single step or a plurality of steps selected from post-treatment steps such as a reprecipitation step, a liquid separation step, an ultrafiltration step, a deprotection step, and a lyophilization step in order to isolate a nucleic acid oligomer. In the storage step, an atmosphere in a storage container may be replaced with an inert gas. Examples of the inert gas include nitrogen gas, argon gas, and helium gas.

In the reprecipitation step, a stabilized solution can be brought into contact with a poor solvent to precipitate and isolate the nucleic acid oligomer. If necessary, the liquid part may be removed from a solid-liquid separated state, followed by collecting and isolating the precipitated nucleic acid oligomer by filtration or the like. Examples of the poor solvent in the reprecipitation step include a C1-C4 organic solvent having at least one oxygen atom (for example, a C1-C4 alcohol, tetrahydrofuran, dioxane). As the solvent, ethanol or isopropanol is preferable.

In the liquid separation step, the stabilized solution is mixed with at least one of an acidic aqueous solution such as an acetic acid aqueous solution, water, and brine and the like, and an organic solvent that is not miscible with water is further added to the stabilized solution to separate the resulting mixture to an aqueous layer and an organic layer, so that the aqueous layer containing a desired nucleic acid oligomer can be obtained.

In the ultrafiltration step, the nucleic acid oligomer present in the solution after the storage step can be separated from low molecular weight components having a desired molecular weight or less using an ultrafiltration membrane.

When a protecting group is present at the 5' position in the end of the nucleic acid oligomer, in order to deprotect the protecting group, the protecting group of the nucleic acid oligomer can be deprotected by mixing the solution after the storage step with an acidic aqueous solution such as an aqueous acetic acid solution or a solution obtained by dissolving an acidic substance such as acetic acid in an organic solvent.

In the lyophilization step, water is sublimated by depressurizing the frozen aqueous solution of the nucleic acid oligomer, and the nucleic acid oligomer and moisture can be separated from each other.

In the synthesis of the nucleic acid oligomer by a phosphoramidite method, a nucleic acid elongation reaction can be performed according to a known method (for example, a method described in JP 5157168 B2 or JP 5554881 B2). Regarding the production of the nucleic acid oligomer by the phosphoramidite method, a method for producing the nucleic acid oligomer will be described with reference to the following reaction route (e.g. coupling reaction, oxidation, deprotection), taking the synthesis of RNA of the scheme shown in Fig. 1 as an example.

In the chemical formula indicating the reaction route, $B^a$ represents a nucleobase which may be protected, Tr represents a protecting group, X is as defined above, and SP represents a portion other than the nucleoside structure

of an inorganic porous carrier.

A nucleobase constituting an inorganic porous carrier having a nucleoside structure (Sp-Nu) and a nucleoside of an amidite monomer (Am-1) is the nucleobase as described above or a nucleobase protected with a protecting group.

Suitable examples of the amidite monomer (Am-1) include TBDMS amidite (TBDMS RNA Amidites, trade name, ChemGenes Corporation), ACE amidite, TOM amidite, CEE amidite, CEM amidite, TEM amidite (Reviews by Chakhmakhcheva: Protective Groups in the Chemical Synthesis of Oligoribonucleotides, Russian Journal of Bioorganic Chemistry, 2013, Vol. 39, No. 1, pp. 1-21), and EMM amidite (as described in WO2013/027843 A), and the like, in which when R represents a protected hydroxy group in a compound represented by the following chemical formula (Am-1'), the specific protecting group is a tert-butyldimethylsilyl (TBDMS) group, a bis(2-acetoxy)methyl (ACE) group, a (triisopropylsilyloxy)methyl (TOM) group, a (2-cyanoethoxy)ethyl (CEE) group, a (2-cyanoethoxy)methyl (CEM) group, a para-tolylsulfonylethoxymethyl (TEM) group, or a (2-cyanoethoxy)methoxymethyl (EMM) group or the like.

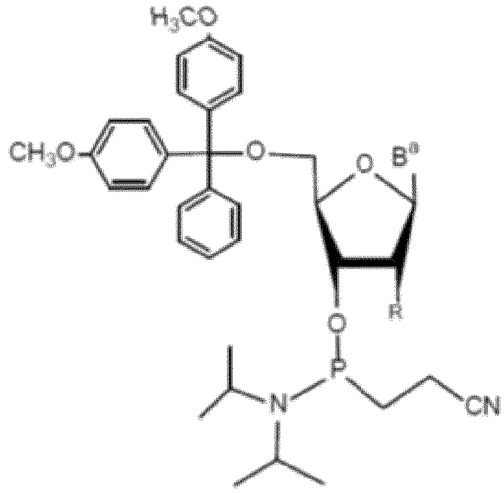

(Am-1')

(wherein R represents the group as described above and $B^a$ represents a nucleobase which may be protected).

[Solid phase synthesis of RNA]

A Tr group of the inorganic porous carrier (Sp-Nu) is deprotected to obtain a solid phase carrier (Am-2). Thereafter, the amidite monomer (Am-1) and the solid phase carrier (Am-2) are subjected to a coupling reaction to obtain a reaction product (Am-3). Thereafter, the reaction product (Am-3) is oxidized to obtain a product (Am-4). Thereafter, the product (Am-4) is deprotected (-Tr) to obtain a product (Am-5). Next, the amidite monomer (Am-1) and the product (Am-5) are further subjected to a coupling reaction to elongate a phosphodiester bond. As described above, the hydroxy group at the 5' position at the end of the elongated oligonucleotide chain is repeatedly subjected to a series of cycle including deprotection, coupling reaction, and oxidation as many times as necessary so as to provide a desired sequence, and then the resulting product can be cleaved from the solid phase carrier to produce a nucleic acid molecule having a desired sequence. The synthesis may be performed using an automated nucleic acid synthesizer or the like employing the phosphoramidite method. Here, RNA will be described as an example, but the present invention can also be applied to a nucleic acid compound containing nucleotide other than ribonucleotide.

In the step of deprotecting a Tr group, the protecting group of the hydroxy group at the 5' position at the end of the RNA chain supported on the solid phase carrier is deprotected. As the protecting group, a trityl-based protecting group (typically, a 4,4'-dimethoxytrityl group (DMTr group)) is used. The deprotection can be performed using an acid. Examples of the acid for deprotection include trifluoroacetic acid, trichloroacetic acid, dichloroacetic acid, trifluoromethanesulfonic acid, methanesulfonic acid, hydrochloric acid, acetic acid, and p-toluenesulfonic acid.

In the coupling step, a nucleoside phosphoramidite is bonded to the hydroxy group at the 5' position in the end of the RNA chain deprotected in the deprotection step so as to produce a phosphite. As the nucleoside phosphoramidite, a nucleoside phosphoramidite in which the hydroxy group at the 5' position is protected by a protecting group (for example, DMTr group) is used.

The coupling step can be performed using an activator which activates the nucleoside phosphoramidite. Examples of the activator include 5-benzylthio-1H-tetrazole (BTT), 1H-tetrazole, 4,5-dicyanoimidazole (DCI), 5-ethylthio-1H-tetrazole (ETT), N-methylbenzimidazolium triflate (N-MeBIT), benzimidazolium triflate (BIT), N-phenylimidazolium triflate (N-PhIMT), imidazolium triflate (IMT), 5-nitrobenzimidazolium triflate (NBT), 1-hydroxybenzotriazole (HOBT), and 5-(bis-3,5-trifluoromethylphenyl)-1H-tetrazole (Activator-42).

After the coupling step, an unreacted hydroxyl group at the 5' position may be capped as appropriate. The capping can be performed using a known capping solution such as an acetic anhydride-tetrahydrofuran solution or a phenoxyacetic anhydride/N-methylimidazole solution.

The oxidation step is a step of oxidizing the phosphite formed in the coupling step. The oxidation step can be performed using an oxidizing agent. Examples of the oxidizing agent include iodine, m-chloroperbenzoic acid, tert-butyl hydroperoxide, 2-butanone peroxide, bis(trimethylsilyl)peroxide, 1,1-dihydroperoxycyclododecane, and hydrogen peroxide.

In the case of converting a phosphite triester group to a thiophosphate triester group, as the "oxidizing agent", for example, sulfur, 3H-1,2-benzodithiol-3-one-1,1-dioxide (Beaucage reagent), 3-amino-1,2,4-dithiazole-5-thione (ADTT), 5-phenyl-3H-1,2,4-dithiazole-3-one (POS), [(N,N-dimethylaminomethylidene)amino]-3H-1,2,4-dithiazoline-3-thione (DDTT), and phenyl acetyl disulfide (PADS) can be used. The oxidizing agent can be used by being diluted with an appropriate solvent so as to have a concentration of 0.001 to 2 M. The solvent used in the reaction is not particularly limited as long as it is not involved in the reaction, and examples thereof include dichloromethane, acetonitrile, pyridine, or any mixed solvent of two or more kinds of solvents at any ratio thereof.

The oxidation step may be performed after the capping procedure, or conversely, the capping procedure may be performed after the oxidation step, and the order of the procedures is not limited.

After the oxidation step, the process returns to the deprotection step, and the above series of steps including coupling reaction, oxidation, and deprotection are repeatedly performed according to the nucleotide sequence of the nucleic acid oligomer to be synthesized, whereby RNA having a desired sequence can be synthesized.

After the synthesis of the nucleic acid oligomer having a desired sequence is completed, the RNA chain is cleaved and recovered from the solid phase carrier using ammonia or an amine compound.

Examples of the amine compound here include methylamine, ethylamine, isopropylamine, ethylenediamine, diethylamine, and triethylamine.

The chain length of the nucleic acid oligomer thus obtained is, for example, within a range of $n \geq 60$, $n \geq 80$, or $n \geq 100$, and $n \leq 200$. The chain length of the nucleic acid oligomer is preferably within a range of $n \geq 60$. The chain length of the nucleic acid oligomer is specifically, for example, within a range of n=67, 100, or 120.

In the step of deprotecting the phosphate protecting group, after the synthesis of the nucleic acid having a desired sequence is completed, an amine compound is allowed to react in order to deprotect the protecting group of the phosphate moiety. Examples of the amine compound include diethylamine as described herein.

In the case where a protecting group for a hydroxy group at the 2' position or 3' position of ribose is present, the protecting group can be removed according to the method described in WO 2006/022323 A1), WO 2013/027843 A1, or WO 2019/208571 A1.

## EXAMPLES

Hereinafter, the present invention will be described in more detail with reference to Examples, but the present invention is not limited to these Examples.

### Measurement method

Each measurement method used in the following test is shown below.

(Measurement method 1: Method for measuring purity of RNA)

The purity of RNA in a solution after fractionation was measured by HPLC. The fractionated RNA was separated into components by HPLC (wavelength 260 nm, column DNAPac™ PA200, 4.0 mm×250 mm, 8.0 pm), and the purity of RNA was calculated from the area value of the peak of a main product in the total area value of the peaks of an obtained chromatogram. A HPLC measurement condition is shown in the following Table 1.

[Table 1]

| | |
|---|---|
| Column | DNAPac™, PA200, 4.0 mm × 250 mm, 8.0 μm |
| Flow rate | 1.0 mL/min |
| Detection wavelength | 260 nm |
| Mobile phase A | 25 mM Tris-HCl Buffer solution (pH 8.0) (Containing 10% acetonitrile, 6M urea) |

(continued)

| | |
|---|---|
| Column | DNAPac™, PA200, 4.0 mm × 250 mm, 8.0 μm |
| Mobile phase B | 25 mM Tris-HCl Buffer solution (pH 8.0) (Containing 500mM sodium perchlorate, 10% acetonitrile, 6M urea) |
| Gradient condition | Bconc. 20% (0 min)-60% (60 min)-90% (60.01 min)-90% (65 min)-20% (65.01 min)-20% (80 min) |
| Column temperature | 80°C |

Reference Example 1

1. Solid phase synthesis of RNA by amidite method

RNA having a nucleic acid sequence of chain I shown below was synthesized. The chain consists of 103 base lengths.

Chain I:

A*U*A*ACUCAAUUUGUAAAAAAGUUUUAGAGCUAGAAAUAGCAAGUUAAAAUAAGGCU

AGUCCGUUAUCAACUUGAAAAAGUGGCACCGAGUCGGUGCUUUU*U*U*U (5'-3')

(Sequence No. 1)

In the notation of the sequence, symbols * between nucleotides represent that a phosphate bond connecting nucleotides is phosphorothioate.

The RNA was synthesized from the 3' side to the 5' side using a nucleic acid synthesizer (AKTA oligopilot plus 100 GE Healthcare) according to a phosphoramidite method. The synthesis was carried out on a 63 μmol scale. Also for the synthesis, an uridine EMM amidite (described in Example 2 of WO 2013/027843 A1), a cytidine EMM amidite (described in Example 3 of the same), an adenosine EMM amidite (described in Examples 4 of the same), and a guanosine EMM amidite (described in Example 5 of the same), each of which is represented by the following formula respectively were used as RNA amidites. Porous glass was used as a solid phase carrier, and a solution of dichloroacetic acid in toluene was used as a deblocking solution. 5-benzylthio-1H-tetrazole was used as a coupling agent, and an iodine solution was used as an oxidizing agent. 3-amino-1,2,4-dithiazole-5-thione was used as a sulfurizing agent, and a phenoxyacetic anhydride solution and an N-methyl imidazole solution were used as a capping solution. After completion of the nucleic acid elongation, a cyanoethyl protecting group of a phosphate moiety was selectively deprotected by allowing a diethylamine solution to act on a nucleic acid on a carrier. Here, EMM is an abbreviation of a (2-cyanoethoxy)methoxymethyl group.

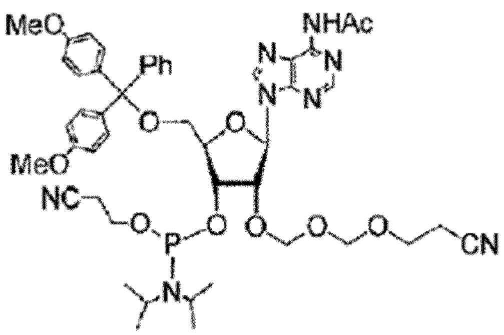

ADENOSINE EMM AMIDITE

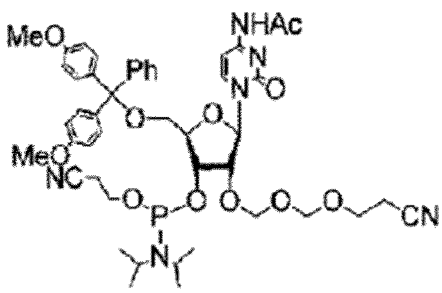

CYTIDINE EMM AMIDITE

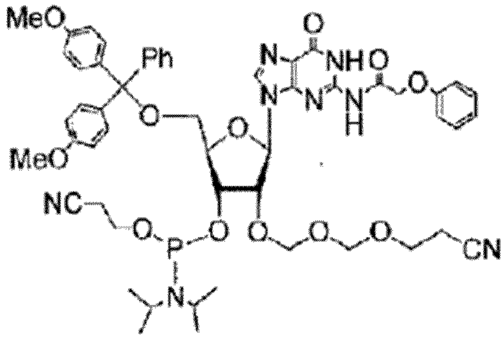

GUANOSINE EMM AMIDITE

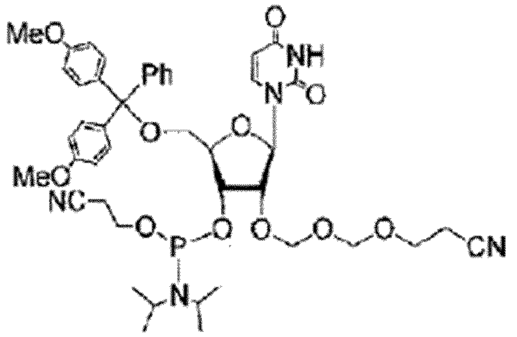

URIDINE EMM AMIDITE

Cleavage from the solid phase carrier and deprotection after solid phase synthesis were performed according to the method described in WO 2013/027843 A1. That is, an aqueous ammonia solution and ethanol were added to the solid phase carrier, and the solid phase carrier was left to stand for a while. Then, the solid phase carrier was filtered to distill off the solvent. Thereafter, the deprotection of a hydroxyl group was performed using tetrabutylammonium fluoride. The obtained RNA was dissolved at a desired concentration using distilled water for injection.

2. Preparative Purification of RNA

Column chromatography purification was performed under the condition of the following Table 2. However, before the purification, a mobile phase A was passed through a column at a flow rate of 4.7 mL/min for 12.5 min, and then the sample was added. The fractions at a retention time of 94.2 min to 95.8 min were collected, and the obtained solutions were analyzed by HPLC. The purity thereof was calculated by the method described in the measurement method 1. As a result, the purity was 87.7%. Using an RNA solution obtained by the preparative purification, the following experiments of Examples and Comparative Examples were performed.

[Table 2]

| | |
|---|---|
| Instrument | AKTApurifier100 (manufactured by GE Healthcare) |
| Detector | UV 260 nm |
| Column | Manufactured by YMC Triart C18 10 mm × 250 mm, 10 μm |
| Separation mode | Reverse phase chromatography |
| Column temperature | 60°C |
| Flow rate | 4.7 mL/min |
| Mobile phase A | 100mM dibutylammonium acetate solution (pH 7.0) |
| Mobile phase B | Acetonitrile |
| Preparative width | 3.9 mL |
| Gradient condition | B conc. 0% (0 min)-30% (12.5 min)-40% (95.8 min)-100% (95.9 min)-100% (104 min) |

Example 1

In a polypropylene vial (Thermo Fisher Scientific Inc.) having a volume of 300 mL, 99 μL of the RNA solution preparatively purified by reverse phase column chromatography in Reference Example 1 was placed, and the solution was mixed with 1 μL of a solution of triphenylphosphine in acetonitrile as an additive solution to prepare a sample having a predetermined concentration. The vial containing the mixed solution was placed in an incubator (Kenis Limited) in which the temperature was adjusted to 60°C, and left to stand for 8 hours. After left to stand, the headspace vial taken out from the incubator was cooled to room temperature, and the purity thereof was calculated by the method described in the above-mentioned measurement method 1. The results are shown in Table 3.

The composition prepared with the concentration of the triphenylphosphine of 3 mM (0.09%) has, on the basis of the calculation, the following composition: water: 63.79%, acetonitrile: 34.87 %, dibutylamine 0.84%, acetic acid: 0.39% (1.23% as dibutylammonium acetate); and nucleic acid concentration: 0.21 mg/mL (0.02%).

Comparative Example 1

In a polypropylene vial (Thermo Fisher Scientific Inc.) having a volume of 300 mL, 100 μL of the RNA solution preparatively purified by reverse phase column chromatography in Reference Example 1 was placed. The vial containing the solution was placed in an incubator (manufactured by Kenis Limited) in which the temperature was adjusted to 60°C, and left to stand for 8 hours. After left to stand, the headspace vial taken out from the incubator was cooled to room temperature, and the purity thereof was calculated by the method described in the above-mentioned measurement method 1. The results are shown in Table 3.

[Table 3]

| | Additive | Concentration of additive (mM) | HPLC measurement purity1) | Retention ratio of nucleic acid2) |
|---|---|---|---|---|
| Example 1 | Triphenylphosphine | 3 | 83.5% | 95.2% |
| Comparative Example 1 | None | | 66.6% | 75.9% |

1) which represents the purity of nucleic acid obtained by leaving the nucleic acid (purity 87.7%) prepared in Reference Example 1 to stand at 60°C for 8 hours.

2) Retention ratio of nucleic acid (%) = Nucleic acid purity after left to stand/Nucleic acid purity before left to stand.

[Reference Example 2]

Solid phase synthesis of RNA by amidite method

RNA having a nucleic acid sequence of chain II represented below was synthesized. The chain consists of 67 base lengths.

Chain II:

Am*Gm*Cm*AmUmAmGmCAAGUUAmAAAUAAGGmC*U*AmG*U*C*CmGUUAUCAAmCm

UmUmGmAmAmAmAmAmGmUmGGCACmCmGmAGUCGGmUmGmCm*Um*Um*U (5'-3')

(Sequence No. 2)

In the notation of the sequence, symbols * between nucleotides represent that a phosphate bond connecting nucleotides is phosphorothioate. Alphabets Am, Um, Cm, and Gm represent a nucleotide wherein a 2' hydroxy group is substituted with a methoxy group. The RNA was synthesized from the 3' side to the 5' side using a nucleic acid synthesizer (AKTA oligopilot plus 100 GE Healthcare) according to a phosphoramidite method. The synthesis was carried out on a 53 pmol scale. Also for the synthesis, an uridine EMM amidite (described in Example 2 of WO 2013/027843 A), a cytidine EMM amidite (described in Example 3 of the same), an adenosine EMM amidite (described in Examples 4 of the same), and a guanosine EMM amidite (described in Example 5 of the same), and an uridine 2'OMe amidite, a cytidine 2'OMe amidite, an adenosine 2'OMe amidite, and a guanosine 2'OMe amidite each of which is represented by the following formula respectively were used as RNA amidites. Porous glass was used as a solid phase carrier, and a solution of dichloroacetic acid in toluene was used as a deblocking solution. 5-benzylthio-1H-tetrazole was used as a coupling agent, an iodine solution was used as an oxidizing agent, and 3-amino-1,2,4-dithiazole-5-thione was used as a sulfurizing agent. A phenoxyacetic anhydride solution and an N-methyl imidazole solution were used as a capping solution. After completion of the nucleic acid elongation, a cyanoethyl protecting group of a phosphate moiety was selectively deprotected by allowing a diethylamine solution to act on a nucleic acid on a carrier. Here, EMM is an abbreviation of a (2-cyanoethoxy)methoxymethyl group.

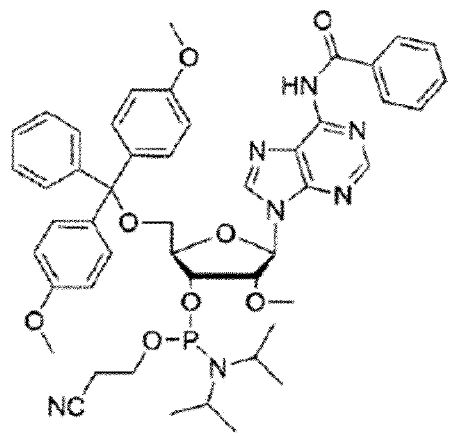

ADENOSINE 2'OME AMIDITE

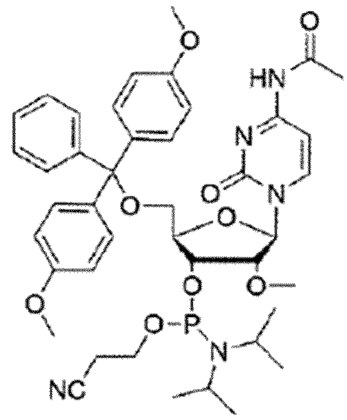

CYTIDINE 2'OME AMIDITE

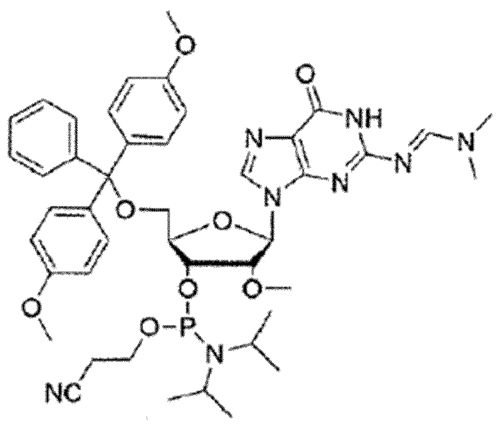

GUANOSINE 2'OME AMIDITE

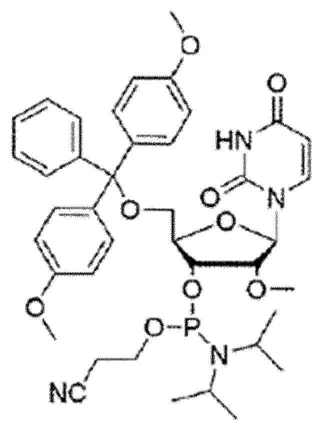

URIDINE 2'OME AMIDITE

Cleavage from the solid phase carrier and deprotection after solid phase synthesis were performed according to the method described in WO 2013/027843 A1. That is, an aqueous ammonia solution and ethanol were added to the solid phase carrier, and the solid phase carrier was left to stand for a while. Then, the solid phase carrier was filtered to distill off the solvent. Thereafter, the deprotection of a hydroxyl group was performed using tetrabutylammonium fluoride. The obtained RNA was dissolved at a desired concentration using distilled water for injection.

Preparative Purification of RNA

Column chromatography purification was performed under the condition of the following Table 4. However, before the purification, a mobile phase A was passed through a column at a flow rate of 4.7 mL/min for 12.5 min, and then the sample was added. The fractions at a retention time of 66.7 min to 70.9 min were collected, and the obtained solutions were analyzed by HPLC. The purity thereof was calculated by the method described in the measurement method 1. As a result, the purity was 94.2%. Using an RNA solution obtained by the preparative purification, the following experiments of Examples and Comparative Examples were performed.

[Table 4]

| | |
|---|---|
| Instrument | AKTApurifier100 (manufactured by GE Healthcare) |
| Detector | UV 260 nm |
| Column | Manufactured by YMC Triart C18 10 mm × 250 mm, 10 μm |
| Separation mode | Reverse phase chromatography |

(continued)

| | |
|---|---|
| Instrument | AKTApurifier100 (manufactured by GE Healthcare) |
| Column temperature | 60°C |
| Flow rate | 4.7 mL/min |
| Mobile phase A | 100 mM dibutylammonium acetate solution (pH 7.0) |
| Mobile phase B | Acetonitrile (containing 10% methanol (v/v)) |
| Preparative width | 3.9 mL |
| Gradient condition | B conc. 0% (0 min)-35% (12.5 min)-50% (137.6 min)-100% (137.7 min)-100% (145.9 min) |

[Example 2]

In a polypropylene vial (Thermo Fisher Scientific Inc.) having a volume of 300 mL, 99 μL of the RNA solution preparatively purified by reverse phase column chromatography in Reference Example 2 was placed, and the solution was mixed with 1 μL of a solution of triphenylphosphine in acetonitrile as an additive solution to prepare a sample having a triphenylphosphine concentration of 3 mM. The vial containing the mixed solution was placed in an incubator (Kenis Limited) in which the temperature was adjusted to 60°C, and left to stand for 8 hours. After left to stand, the polypropylene vial taken out from the incubator was cooled to room temperature, and the purity of the solution was calculated by the method described in the above-mentioned measurement method 1. The results are shown in Table 5.
The composition prepared with the concentration of the triphenylphosphine of 3 mM (0.09%) has, on the basis of the calculation, the following composition: water: 62.02%, acetonitrile: 33.06%, methanol: 3.60%, dibutylamine: 0.82%, acetic acid: 0.38% (1.20% as dibutylammonium acetate); and nucleic acid concentration: 0.31 mg/mL (0.03%).

[Example 3]

An experiment was performed under the same conditions except that a solution of diethyl phosphite in acetonitrile was used instead of the solution of triphenylphosphine in acetonitrile as the additive solution in the experiment of Example 2 to prepare a solution having a diethyl phosphite concentration of 3 mM (0.05%), and the purity of RNA after the experiment was measured. The results are shown in Table 5.

[Example 4]

An experiment was performed under the same conditions except that a solution of triethyl phosphite in acetonitrile was used instead of the solution of triphenylphosphine in acetonitrile as the additive solution in the experiment of Example 2 to prepare a solution having a triethyl phosphite concentration of 3 mM (0.05%), and the purity of RNA after the experiment was measured. The results are shown in Table 5.

[Example 5]

An experiment was performed under the same conditions except that a solution of diethoxyphenylphosphine in acetonitrile was used instead of the solution of triphenylphosphine in acetonitrile as the additive solution in the experiment of Example 2 to prepare a solution having a diethoxyphenylphosphine concentration of 3 mM (0.07%), and the purity of RNA after the experiment was measured. The results are shown in Table 5.

[Example 6]

An experiment was performed under the same conditions except that a solution of ethoxydiphenylphosphine in acetonitrile was used instead of the solution of triphenylphosphine in acetonitrile as the additive solution in the experiment of Example 2 to prepare a solution having an ethoxydiphenylphosphine concentration of 3 mM (0.08%), and the purity of RNA after the experiment was measured. The results are shown in Table 5.

[Example 7]

An experiment was performed under the same conditions except that a solution of 9,10-dihydro-9-oxa-10-phosphaphenanthrene 10-oxide in acetonitrile was used instead of the solution of triphenylphosphine in acetonitrile as

the additive solution in the experiment of Example 2 to prepare a solution having a 9,10-dihydro-9-oxa-10-phosphaphenanthrene 10-oxide concentration of 3 mM (0.07%), and the purity of RNA after the experiment was measured. The results are shown in Table 5.

[Comparative Example 2]

In a polypropylene vial (Thermo Fisher Scientific Inc.) having a volume of 300 mL, 100 μL of the RNA solution preparatively purified by reverse phase column chromatography in Reference Example 2 was placed, and the vial containing the solution was placed in an incubator (manufactured by Kenis Limited) in which the temperature was adjusted to 60°C, and left to stand for 8 hours. After left to stand, the polypropylene vial taken out from the incubator was cooled to room temperature, and the purity of the solution was calculated by the method described in the above-mentioned measurement method 1. The results are shown in Table 5.

[Table 5]

| | Additive | Concentration of additive (mM) | HPLC measurement purity[1)] | Retention ratio of nucleic acid[2)] |
|---|---|---|---|---|
| Example 2 | Triphenylphosphine | 3 | 93.1% | 98.8% |
| Example 3 | Diethyl phosphite | 3 | 86.4% | 91.7% |
| Example 4 | Triethyl phosphite | 3 | 92.3% | 98.0% |
| Example 5 | Diethoxyphenylphosphine | 3 | 92.3% | 98.0% |
| Example 6 | Ethoxydiphenylphosphine | 3 | 89.4% | 94.9% |
| Example 7 | 9,10-dihydro-9-oxa-10-phosphaphenanthrene 10-oxide | 3 | 89.4% | 94.9% |
| Comparative Example 2 | None | | 82.4% | 87.5% |

1) which represents the purity of the nucleic acid obtained by leaving the nucleic acid (purity 94.2%) prepared in the Reference Example 2 to stand at 60°C for 8 hours.
2) Retention ratio of nucleic acid (%) = Nucleic acid purity after left to stand/Nucleic acid purity before left to stand.

[Reference Example 3]

Preparative Purification of RNA

The RNA with a hydroxy group being deprotected using tetrabutylammonium fluoride obtained in Reference Example 2 was subjected to column chromatography purification under the conditions of the following Table 6. However, before the purification, a mobile phase A was passed through a column at a flow rate of 4.7 mL/min for 12.5 min, and then the sample was added. The fractions at a retention time of 91.7 min to 94.2 min were collected, and the obtained solutions were analyzed by HPLC. The purity thereof was calculated by the method described in the measurement method 1. As a result, the purity was 95.1%. Using an RNA solution obtained by the preparative purification, the following experiments of Examples and Comparative Examples were performed.

[Table 6]

| | |
|---|---|
| Instrument | AKTApurifier100 (manufactured by GE Healthcare) |
| Detector | UV 260 nm |
| Column | manufactured by YMC Triart C18 10 mm × 250 mm, 10 μm |
| Separation mode | Reverse phase chromatography |
| Column temperature | 60°C |
| Flow rate | 4.7 mL/min |
| Mobile phase A | 100mM aqueous hexylammonium acetate solution (pH 7.0) |

(continued)

| | |
|---|---|
| Instrument | AKTApurifier100 (manufactured by GE Healthcare) |
| Mobile phase B | Acetonitrile (containing 10% methanol (v/v)) |
| Preparative width | 3.9 mL |
| Gradient condition | B conc. 0% (0 min)-35% (12.5 min)-50% (137.6 min)-100% (137.7 min)-100% (145.9 min) |

[Example 8]

In a polypropylene vial (Thermo Fisher Scientific Inc.) having a volume of 300 mL, 99 μL of the RNA solution preparatively purified by reverse phase column chromatography in Reference Example 3 was placed, and the solution was mixed with 1 μL of a solution of triphenylphosphine in acetonitrile as an additive solution to prepare a sample having a triphenylphosphine concentration of 3 mM. The vial containing the mixed solution was placed in an incubator (Kenis Limited) in which the temperature was adjusted to 60°C, and left to stand for 14 hours. After left to stand, the polypropylene vial taken out from the incubator was cooled to room temperature, and the purity of the solution was calculated by the method described in the above-mentioned measurement method 1. The results are shown in Table 7.

The composition prepared with the concentration of the triphenylphosphine of 3.0 mM (0.09%) has, on the basis of the calculation, the following composition: water: 59.70%, acetonitrile: 35.36%, methanol: 3.84%, hexylamine: 0.61%, acetic acid: 0.36% (0.97% as hexylammonium acetate), and nucleic acid concentration: 0.35 mg/mL (0.04%).

[Comparative Example 3]

In a polypropylene vial (Thermo Fisher Scientific Inc.) having a volume of 300 mL, 100 μL of the RNA solution preparatively purified by reverse phase column chromatography in Reference Example 3 was placed, and the vial containing the solution was placed in an incubator (manufactured by Kenis Limited) in which the temperature was adjusted to 60°C, and left to stand for 14 hours. After left to stand, the polypropylene vial taken out from the incubator was cooled to room temperature, and the purity of the solution was calculated by the method described in the above-mentioned measurement method 1. The results are shown in Table 7.

[Table 7]

| | Additive | Concentration of additive (mM) | HPLC measurement purity[1)] | Retention ratio of nucleic acid[2)] |
|---|---|---|---|---|
| Example 8 | Triphenylphosphine | 3 | 91.9% | 96.6% |
| Comparative Example 3 | None | | 88.8% | 93.4% |
| 1) which represents the purity of the nucleic acid obtained by leaving the nucleic acid (purity 95.1%) prepared in Reference Example 2 to stand at 60°C for 14 hours. | | | | |

Retention ratio of nucleic acid (%) = Nucleic acid purity after left to stand/Nucleic acid purity before left to stand.

[Example 9] (Recovery of RNA from preparatively purified RNA solution)

In Example 8, a solution obtained by mixing triphenylphosphine to adjust the concentration thereof to 3 mM followed by leaving to stand at 60°C for 14 hours was subjected to the following treatment. In a polypropylene conical tube (Corning Inc.) having a volume of 15 mL, 60 μL of the solution was placed, and 30 μL of an aqueous sodium acetate solution (3 M, pH = 5.2) and 180 μL of ethanol were added thereto. The resulting slurry solution was centrifuged with 3000 g at 25°C for 10 min to remove the supernatant. Subsequently, 150 μL of a 70% aqueous ethanol solution was placed in the tube, the mixture was centrifuged with 3000 g at 25°C for 10 min to remove the supernatant. These procedures were repeatedly carried out twice to obtain RNA. The obtained RNA was solubilized in 60 μL of water, and the RNA purity in the fraction was calculated by the method described in the above-mentioned measurement 1 to indicate

the purity of 93.1%.

[Comparative Example 4]

The solution left to stand at 60°C for 14 hours in Comparative Example 3 was subjected to the following treatment. In a polypropylene conical tube (Corning Inc.) having a volume of 15 mL, 60 μL of the solution was placed, and 30 μL of an aqueous sodium acetate solution (3 M, pH = 5.2) and 180 μL of ethanol were added thereto. The resulting slurry solution was centrifuged with 3000 g at 25°C for 10 min to remove the supernatant. Subsequently, 150 μL of a 70% aqueous ethanol solution was placed in the tube, the mixture was centrifuged with 3000 g at 25°C for 10 min to remove the supernatant. These procedures were repeatedly carried out twice to obtain RNA. The obtained RNA was solubilized in 60 μL of water, and the RNA purity in the fraction was calculated by the method described in the above-mentioned measurement 1 to indicate the purity of 83.0%.

INDUSTRIAL APPLICABILITY

According to the method of the present invention, a nucleic acid oligomer having a phosphorothioate bond can be stabilized and efficiently produced.

SEQUENCE LISTING FREE TEXT

SEQ ID NOs: 1 and 2 in the Sequence Listing represent the base sequences of oligonucleotides produced according to the production method of the present invention.

## Claims

**1.** A composition comprising a nucleic acid oligomer having a phosphorothioate bond represented by Formula (1):

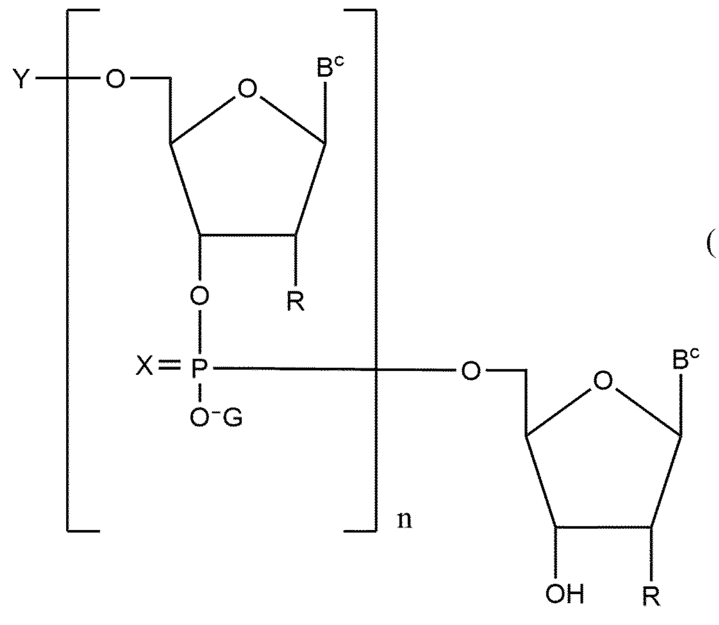

(1)

wherein:

$B^C$ each independently represents the same nucleobase or different nucleobases,
R is the same or different from each other and each independently represents a hydrogen atom, a fluorine atom, or OQ group,
Q is the same or different from each other and each independently represents a hydrogen atom, a methyl group, a 2-methoxyethyl group, a methylene group bonded to a carbon atom at a 4'-position of ribose, an ethylene group bonded to a carbon atom at a 4'-position of ribose, or an ethylidene group bonded to a carbon atom at a 4'-position of ribose,

X is the same or different from each other and each independently represents an oxygen atom or a sulfur atom wherein at least one X represents a sulfur atom,
Y represents a hydrogen atom or a protecting group of a hydroxy group,
G represents an ammonium ion, an alkylammonium ion, an alkali metal ion, a hydrogen ion or a hydroxyalkylammonium ion, and
n is an integer satisfying Formula (2):

$$15 \leq n \quad (2),$$

an alkyl ammonium salt, a nitrile-based organic solvent, water, and an additive,
wherein the additive is an additive containing at least one compound selected from the group consisting of compounds represented by the following Formulae (3a) to (3h) :

Formula (3a): $P(L^1)_3$,

Formula (3b): $P(OL^1)_3$,

Formula (3c) : $P(L^1)_2(OL^1)$,

Formula (3d): $PL(OL^1)_2$,

Formula (3e): $PH(O)(OL^1)_2$,

Formula (3f): $PH(O)L^1(OL^1)$,

Formula (3g) : $(P(L^1)_2)_2 - L^2$,

Formula (3h):

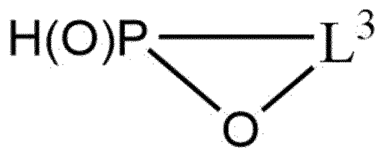

,

wherein, in each of Formulae (3a) to (3h), $L^1$ is the same or different from each other and each independently represents a C6-10 aryl group which may be substituted with 1 to 3 substituents selected from the group consisting of a C1-6 alkyl group and a C1-6 alkoxy group, or a C1-6 alkyl group,
$L^2$ is the same or different from each other and each independently represents a C6-14 arylene group which may be substituted with 1 to 3 substituents selected from the group consisting of a C1-6 alkyl group and a C1-6 alkoxy group, or a C1-6 alkylene group, and
$L^3$ is the same or different from each other and each independently represents a C6-14 arylene group which may be substituted with 1 to 3 substituents selected from the group consisting of a C1-6 alkyl group and a C1-6 alkoxy group.

**2.** The composition according to claim 1, wherein the additive is at least one compound selected from the group consisting of triphenylphosphine, diethyl phosphite, triethyl phosphite, diethoxyphenylphosphine, ethoxydiphenylphosphine, and 9,10-dihydro-9-oxa-10-phosphaphenanthrene 10-oxide.

**3.** The composition according to any one of claims 1 and 2, wherein the alkyl ammonium salt is at least one alkyl ammonium salt selected from the group consisting of a monoalkylammonium salt and a dialkylammonium salt.

**4.** The composition according to any one of claims 1 to 3, wherein the nitrile-based organic solvent is acetonitrile.

5. The composition according to any one of claims 1 to 4, wherein in Formula (1), R is each independently a hydroxy group or a methoxy group.

6. A method for producing a nucleic acid oligomer, comprising mixing the composition according to any one of claims 1 to 5 with at least one solvent selected from the group consisting of a C1-C4 alcohol, tetrahydrofuran and dioxane to isolate a precipitated nucleic acid oligomer.

7. A method for producing the composition according to any one of claims 1 to 5, comprising mixing a column eluate containing a nucleic acid oligomer represented by Formula (1) wherein the nucleic acid oligomer is obtained by subjecting a crude product of the nucleic acid oligomer of Formula (1) synthesized by a solid phase synthesis method to a reverse phase column chromatography treatment, an alkyl ammonium salt, a water-soluble organic solvent, and water, with an additive.

Fig. 1

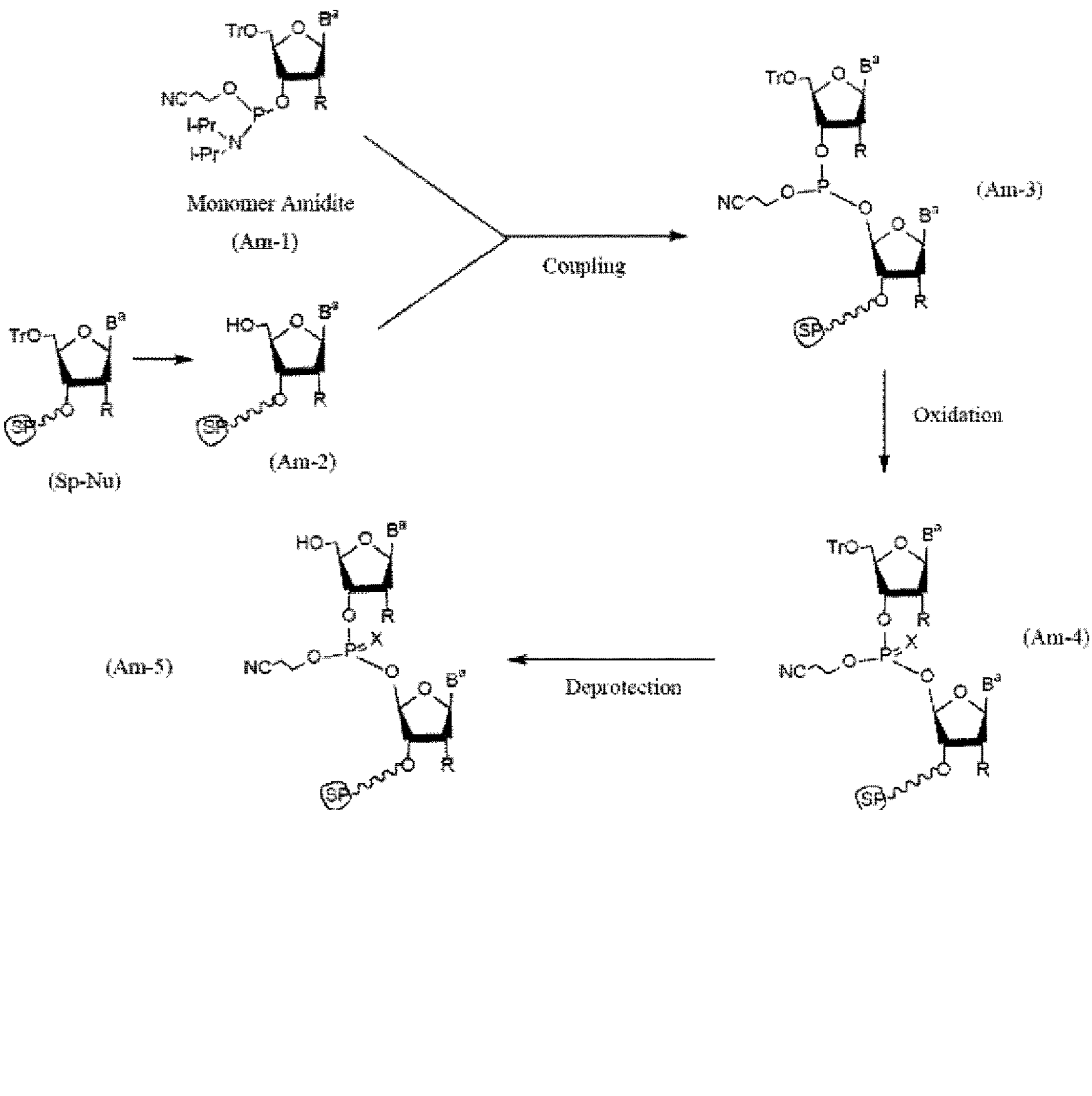


(Am-1) (Sp-Nu) (Am-2) (Am-3) (Am-4) (Am-5)

**INTERNATIONAL SEARCH REPORT**

International application No.
**PCT/JP2022/025692**

**A. CLASSIFICATION OF SUBJECT MATTER**

***C07H 21/02***(2006.01)i
FI: C07H21/02

According to International Patent Classification (IPC) or to both national classification and IPC

**B. FIELDS SEARCHED**

Minimum documentation searched (classification system followed by classification symbols)

C07H21/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/196890 A1 (AJINOMOTO CO., INC.) 01 October 2020 (2020-10-01) paragraphs [0002], [0072], claims, examples | 1-7 |
| Y | WO 2021/024467 A1 (SUMITOMO CHEMICAL CO., LTD.) 11 February 2021 (2021-02-11) claims, examples | 1-7 |
| Y | WO 2021/024465 A1 (SUMITOMO CHEMICAL CO., LTD.) 11 February 2021 (2021-02-11) claims, examples | 1-7 |
| Y | WO 2017/115655 A1 (SHOWA DENKO KK) 06 July 2017 (2017-07-06) claims, examples | 1-7 |
| Y | WO 2018/212236 A1 (NISSAN CHEMICAL CORP.) 22 November 2018 (2018-11-22) paragraphs [0217], [0220], [0226], [0228], [0229], [0230], claims, examples | 1-6 |
| A | | 7 |
| A | JP 2020-515525 A (GENE SIGNAL INTERNATIONAL S. A.) 28 May 2020 (2020-05-28) claims | 1-7 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 August 2022** | **06 September 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2022/025692**

**C. DOCUMENTS CONSIDERED TO BE RELEVANT**

| Category* | Citation of document, with indication, where appropriate, of the relevant passages | Relevant to claim No. |
|---|---|---|
| P, Y | WO 2021/210409 A1 (SUMITOMO CHEMICAL CO., LTD.) 21 October 2021 (2021-10-21) claims, examples | 1-7 |

Form PCT/ISA/210 (second sheet) (January 2015)

**INTERNATIONAL SEARCH REPORT**

International application No. **PCT/JP2022/025692**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/JP2022/025692**

| Patent document cited in search report | Publication date (day/month/year) | Patent family member(s) | Publication date (day/month/year) |
|---|---|---|---|
| WO 2020/196890 A1 | 01 October 2020 | US 2022/0010348 A1<br>paragraph [0003], claims, examples<br>EP 3950698 A1<br>CN 113631564 A | |
| WO 2021/024467 A1 | 11 February 2021 | (Family: none) | |
| WO 2021/024465 A1 | 11 February 2021 | (Family: none) | |
| WO 2017/115655 A1 | 06 July 2017 | TW 201726917 A | |
| WO 2018/212236 A1 | 22 November 2018 | (Family: none) | |
| JP 2020-515525 A | 28 May 2020 | US 2018/0221401 A1<br>claims<br>US 2019/0350963 A1<br>WO 2018/141908 A1<br>CA 3051724 A1<br>CN 110520134 A | |
| WO 2021/210409 A1 | 21 October 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017068377 A1
- US 3687808 A
- JP 5157168 B
- JP 5554881 B
- WO 2013027843 A
- WO 2006022323 A1
- WO 2013027843 A1
- WO 2019208571 A1


### Non-patent literature cited in the description


- Concise Encyclopedia of Polymer Science and Engineering. John Wiley & Sons, 1990, 858-859
- **ENGLISCH et al.** *Angewandte Chemie, International Edition,* 1991, vol. 30, 613
- **CHAKHMAKHCHEVA.** Protective Groups in the Chemical Synthesis of Oligoribonucleotides. *Russian Journal of Bioorganic Chemistry,* 2013, vol. 39 (1), 1-21